# EUROPEAN PATENT APPLICATION

(11) **EP 2 248 904 A1**
(43) Date of publication of application: **10.11.2010**
(21) Application number: 09159401.0
(22) Date of filing: 05.05.2009
(51) Int. Cl.: C12P 7/18, C12N 1/36

(54) **Continuous culture for 1,3-propanediol production using high glycerine concentration**

(71) Applicant: Metabolic Explorer, 63360 Saint Beauzire (FR)
(72) Inventor: Chateau, Michel, 63200, RIOM (FR); Dubois, Jean-Yves, 63360, SAINT BEAUZIRE (FR); Soucaille, Philippe, 31450, DEYME (FR)
(74) Representative: Tetaz, Franck Claude Edouard

(57) **Abstract**

The present invention concerns a new method for the production of 1,3-propanediol comprising culturing a microorganism on a culture medium with high glycerine content. The invention also concerns a new microorganism, or strain of microorganism, adapted for the production of 1,3-propanediol from a medium comprising high glycerine content.
The invention also concerns an "adapted microorganism" which glycerol metabolism is directed to 1,3-propanediol production, and which is allowed to grow in the presence of a high concentration of industrial glycerine.

## Description

The present invention concerns a new method for the production of 1,3-propanediol comprising culturing a microorganism on a culture medium with high glycerine content. The invention also concerns a new microorganism, or strain of microorganism, adapted for the production of 1,3-propanediol from a medium comprising high glycerine content. The invention also concerns an "adapted microorganism" which glycerol metabolism is directed to 13,-propanediol production, and which is allowed to grow in the presence of a high concentration of industrial glycerine.

### Background of the invention

1,3-Propanediol (PDO) is one of the oldest known fermentation products. It was reliably identified as early as 1881 by August Freund, in a glycerol-fermenting mixed culture obviously containing *Clostridium pasteurianum* as the active organism. Quantitative analysis of the fermentation of different enterobacteria producing PDO (trimethylene glycol, propylene glycol) started at the microbiology school of Delft as early as 1928 and was successfully continued at Ames, Iowa in the 1940s. In the 1960s, interest shifted to the glycerol-attacking enzymes, in particular to the glycerol and diol dehydratases, as these enzymes were peculiar in requiring coenzyme B12. PDO-forming clostridia were first described in 1983 as part of a process to obtain a specialty product from glycerol-excreting algae (Nakas *et al.,* 1983). PDO is a typical product of glycerol fermentation and has not been found in anaerobic conversions of other organic substrates. Only very few organisms, all of them bacteria, are able to form it. They include enterobacteria of the genera *Klebsiella* (*K. pneumoniae*), *Enterobacter* (*E. agglomerans)* and *Citrobacter (C. freundii),* lactobacilli (*L. brevis* and *L. buchneri*) and clostridia of the *C. butyricum* and the *C. pasteurianum* group.

Analysis of the fermentation products shows that part of the glycerol is converted to the same products as in the sugar fermentation of this different species, namely acetic acid, 2,3-butanediol, butyric acid, lactic acid, ethanol and succinic acid. This conversion provides the necessary energy for growth but, for many of the products, reducing equivalents are also released, which are used in a reductive conversion of glycerol to PDO. Butyrate formation, which lowers the PDO yield in clostridia, is somewhat comparable to ethanol formation in *Klebsiella,* but it seems to be more dependent on growth rate. Butyrate decreases rapidly with the dilution rate, even in the absence of substrate excess. In any case, changes in the acetate/butyrate ratio do not have such a great impact on the PDO yield.

PDO, as a bi-functional organic compound, could potentially be used for many synthesis reactions, in particular as a monomer for polycondensations to produce polyesters, polyethers and polyurethanes. PDO can be produced by different chemicals routes but they generate waste streams containing extremely polluting substances and the cost of production is then high and chemically produced PDO could not compete with the petrochemically available, diols like 1,2-ethanediol, 1,2-propanediol, and 1,4-butanediol. Therefore, in the past PDO has only found niche applications of negligible market volume. It is the reason why in 1995 Dupont started a research program for the biological conversion of glucose to PDO. Although this process is environmentally friendly it has the disadvantage to i) use vitamin B12 a very expensive cofactor and ii) be a discontinuous process due to the instability of the producing strain. Due to the availability of large amount of glycerol issued from bio-diesel industry, a continuous process, vitamin B12 free with a higher carbon yield would be advantageous.

It is known in the art that PDO can be produced from glycerine, an unwanted by product of the biodiesel production which contains roughly 80-85% of glycerol mixed with salts and water.

*C. butyricum* was previously described as been able to grow and produce PDO from industrial glycerol in batch and two-stage continuous fermentation (Papanikolaou *et al.,* 2000). However, at the highest glycerol concentration, the maximal PDO titre obtained was 48.1 g/L at a dilution rate of 0.02h-1, meaning a productivity of 0.9 g/L/H. The cultures were conducted with a maximum glycerol concentration in the fed medium of 90g/L and in the presence of yeast extract, a costly compound containing organic nitrogen that is known by the man skilled in the art to help in increasing bacterial biomass production.

WO2006/128381 discloses the use of this glycerine for the production of PDO with batch and Fed-Batch cultures using natural PDO producing organisms such as *Klebsiella pneumoniae, C. butyricum* or *C. pasteuricum..* Furthermore, the medium used in WO2006/128381 also contains yeast extract,. As described in this patent application, the maximal productivity reached is comprised between 0.8 and 1.1g.l.h.

The performance of a *C. acetobutylicum* strain modified to contain the vitamin B12-independent glycerol-dehydratase and the PDO-dehydrogenase from *C. butyricum,* called *C. acetobutylicum* DG1 pSPD5 has been described in Gonzalez-Pajuelo *et al.,* 2005. This strain originally grows and produces PDO with a fed medium containing up to 120g.l of pure glycerol. In addition, analyses with a fed medium of containing a maximum 60g.l of pure or industrial glycerol did not identify any differences. When comparing *C. butyricum* to *C. acetobutylicum* DG1 pSPD5 a global similar behaviour was observed by the authors Gonzalez-Pajuelo *et al.,* 2006.

However, the same *C. acetobutylicum* DG1 pSPD5 strain tested with 105g.l. of industrial glycerol in a synthetic fed medium without organic nitrogen, could not give the same performances as with the same concentration of pure glycerol (see example 1).

The present invention provides means for the production of 1,3-propandeiol with high concentration of industrial glycerine and where a higher PDO titre and productivity can be reached.

### Brief description of the invention

The present invention concerns a method for the production of 1,3-propanediol in a continuous fermentation process of glycerine, comprising culturing a producing microorganism on a culture medium, said producing microorganism allowing the conversion of glycerol into 1,3-propanediol, and recovering the 1,3-propanediol, **characterized in that** the culture medium comprises a high concentration of industrial glycerine and wherein the producing microorganism is a microorganism previously adapted to grow in the presence of a high concentration of industrial glycerine.

In preferred embodiments, the glycerol concentration in the culture medium is comprised between 90 and 120 g/L, more preferably about 105g/L of glycerol.

The culture medium is preferably a synthetic medium, without addition of organic nitrogen.

The industrial glycerine is particularly a by-product from biodiesel production.

The producing microorganism is preferably a bacteria, more preferably selected from members of the genera *Clostridium,* particularly *Clostridium acetobutylicum.*

The producing microorganism is advantageously a genetically modified microorganism to allow improved production of 1,3-propanediol form glycerol.

In a preferred embodiment, the glycerol dehydratase activity in the producing microorganism is independent of the presence of coenzyme B12 or one of its precursors and is derived from *Clostridium butyricum.*

Preferably, the producing microorganism is a microorganism previously adapted to grow on the culture medium having high concentration of industrial glycerine by culturing a microorganism on a culture medium comprising high concentration of industrial glycerine at a low dilution rate and selecting the adapted microorganism.

Eventually, the 1,3-propanediol is further purified.

The present invention also concerns a method for the modification of a microorganism into a microorganism adapted to grow in the presence of a high concentration of industrial glycerine, comprising culturing the microorganism on a culture medium comprising high concentration of industrial glycerine at a low dilution rate and selecting the adapted microorganism able to grow on the culture medium having high concentration of industrial glycerine.

The microorganism is advantageously cultured at low dilution rate over a period ranging from 24 hours to 10 days, preferably more than 2 days, more preferably about 8 days.

The dilution rate is generally comprised between 0,005 and 0,1 h⁻¹, preferably between 0,005 and 0,02 h⁻¹. The dilution rate can be changed during the adaptation method, eventually with a first step comprised between 0,005 and 0,02 h⁻¹ and a second step where the dilution rate is increased up to 0.1 h⁻¹, preferably 0,06 h⁻¹

The present invention also concerns an adapted microorganism, a producing microorganism adapted to grow in the presence of a high concentration of industrial glycerine obtainable by the method as disclosed above and below.

### Detailed description of the invention

The term "microorganism" means a microorganism selected among the group consisting of bacteria, yeast and fungi. Preferentially, the microorganism is a bacterium preferably selected among the group consisting of Enterobacteriaceae, Bacillaceae, Clostridiaceae, Streptomycetaceae and Corynebacteriaceae. More preferentially, the bacterium is selected among the group consisting of *Escherichia sp.* (preferably *Escherichia coli), Bacillus sp.* (preferably *Bacillus subtilis), Clostridium sp.* (preferably *Clostridium acetobutylicum)* and *Corynebacterium sp* (preferably *Corynebacterium glutamicum).*

The terms *"Escherichia", "Bacillus", "Clostridium"* and *"Clostridia" and "Corynebacterium"* refer to all kind of bacteria belonging to this family.

The term "producing microorganism" or "producing strain of microorganism" means a microorganism or a strain of microorganism used in the method of production according to the invention where the glycerol metabolism is directed to 13,-propanediol production.

A "microorganism being adapted" means a microorganism being modified to be allowed to grow on high concentrations of industrial glycerine.

An "appropriate culture medium" or a "culture medium" refers to a culture medium optimized for the growth and the diol-production of the producing strain.

The terms "high glycerine content" or "high concentration of glycerine" means more than 90 g/l of glycerol in the culture medium. In preferred embodiments, the glycerol concentration is comprised between 90 and 120 g/L of glycerine, more preferably about 105g/L of glycerine.

"Industrial glycerine" means a glycerine product obtained from an industrial process without substantial purification. Industrial glycerine can also be designated as "raw glycerine". Industrial glycerine comprises more than about 70% of glycerol, preferably more than about 80 %, water and impurities such as mineral salts and fatty acids.

The maximum content of glycerol in industrial glycerine is generally 90%, more generally about 85%.

Industrial processes form which industrial glycerine is obtained are, inter alia, manufacturing methods where fats and oils, particularly fats and oils of plant origin, are processed into industrial products such as detergent or lubricants. In such manufacturing methods, industrial glycerine is considered as a by-product.

In a particular embodiment, the industrial glycerine is a by-product from biodiesel production and comprises known impurities of glycerine obtained from biodiesel production, comprising about 80 to 85% of glycerol with salts, water and some other organic compounds such as fatty acids. Industrial glycerine obtained from biodiesel production has not been subjected to further purification steps.

The term "synthetic medium" means a culture medium comprising a chemically defined substrate upon which organisms are grown.

In the culture medium of the present invention, glycerol is advantageously the single source of carbon.

The phrase "microorganism is modified" means that the strain has been transformed in the aim to change its genetic characteristics. Endogenous genes can be attenuated, deleted, or over-expressed. Exogenous genes can be introduced, carried by a plasmid, or integrated into the genome of the strain, to be expressed into the cell.

The term "plasmid" or "vector" as used herein refers to an extra chromosomal element often carrying genes which are not part of the central metabolism of the cell, and usually in the form of circular double-stranded DNA molecules.

The term "glycerol pulse" as used herein refers to the addition of an extra glycerol amount between the batch and continuous culture. This addition can be done with pure glycerol or a solution similar in composition to the fed medium, or any glycerol concentration in between.

In the description of the present invention, enzymes are identified by their specific activities. This definition thus includes all polypeptides that have the defined specific activity also present in other organisms, more particularly in other microorganisms. Often enzymes with similar activities can be identified by their grouping to certain families defined as PFAM or COG. PFAM (protein family database of alignments and hidden Markov models; http://pfam.sanger.ac uk/) represents a large collection of protein sequence alignments. Each PFAM makes it possible to visualize multiple alignments, see protein domains, evaluate distribution among organisms, gain access to other databases, and visualize known protein structures. COGs (Clusters of Orthologous Groups of proteins; http://www.ncbi.nlm.nih.gov/COG/) are obtained by comparing protein sequences from 43 fully sequenced genomes representing 30 major phylogenetic lines. Each COG is defined from at least three lines, which permits the identification of former conserved domains.

The means of identifying homologous sequences and their percentage homologies are well known to those skilled in the art, and include in particular the BLAST programs, which can be used from the website http://blast.ncbi.nlm.nih.gov/Blast.cgi with the default parameters indicated on that website. The sequences obtained can then be exploited (e.g., aligned) using, for example, the programs CLUSTALW (http://www.ebi.ac.uk/Tools/clustalw2/index.html) or MULTALIN (http://bioinfo.genotoul.fr/multalin/multalin.html) with the default parameters indicated on those websites. Using the references given on GenBank for known genes, those skilled in the art are able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well known to those skilled in the art, and are described, for example, in Sambrook et al. (1989 Molecular Cloning: a Laboratory Manual. 2nd ed. Cold Spring Harbor Lab., Cold Spring Harbor, New York.).

The present invention concerns a method for the production of 1,3-propanediol in a continuous fermentation process of glycerine, comprising culturing a producing microorganism on a culture medium, said producing strain of microorganism allowing the conversion of glycerine into 1,3-propanediol, and recovering the 1,3-propanediol, **characterized in that** the culture medium comprises a high concentration of industrial glycerine, said glycerine being a by-product from biodiesel production and wherein the producing microorganism is a microorganism previously adapted to grow in the presence of a high concentration of industrial glycerine.

Method for directing the glycerol metabolism towards production of 1,3-propanediol are known in the art (see for instance WO2006/128381, González-Pajuelo & al. 2006)

In one embodiment of the invention, the producing microorganism presents an increased flux of 1,3-propanediol production by introducing extra copies of the 1,3-propanediol operon from *C. butyricum,* (coding for enzymes involved in the vitamin B12-independent 1,3-propanediol pathway) either over-expressed by a plasmid or integrated into the chromosome of the microorganism. For example the pSPD5 plasmid can be used for an over-expression of the 1,3-propanediol operon.

In a preferred embodiment, the microorganism is a *Clostridium* strain, more preferably *Clostridium acetobutylicum.*

Continuous fermentation processes are known to the person skilled in the art.

The fermentation process is generally conducted in reactors with an inorganic culture medium of known defined composition adapted to the bacteria used, containing at least glycerine, a by-product from biodiesel production containing glycerol, and if necessary a co-substrate for the production of the metabolite.

This method of the invention is preferably realized in a continuous process. The man skilled in the art knows how to manage each of these experimental conditions, and to define the culture conditions for the microorganisms according to the invention. In particular the *clostridia* are fermented at a temperature between 20°C and 60°C, preferentially between 25°C and 40°C for *C. acetobutilicum.*

Methods for recovering and eventually purifying 1,3-propanediol from a fermentation medium are known to the skilled person. 1,.3-propanediol may be isolated by distillation. In most embodiment, 1,3-propanediol is distilled from the fermentation medium with a by-product, such as acetate, and then further purified by known methods.

In the method according to the present invention, the producing microorganism is adapted to grow in the presence of a high concentration of industrial glycerine.

Several "adaptation process" may be chosen by the person skilled in the art to transform the producing microorganism into a producing microorganism allowed to grow in the presence of a high concentration of industrial glycerine.

In a preferred embodiment, the modification of a microorganism into a microorganism adapted to grow in the presence of a high concentration of industrial glycerine, comprises culturing the microorganism on a culture medium comprising high concentration of industrial glycerine at a low dilution rate and selecting the adapted microorganism able to grow on the culture medium having high concentration of industrial glycerine.

The microorganism is advantageously cultured at low dilution rate over a period ranging from 24 hours to 10 days, preferably more than 2 days, more preferably about 8 days.

The dilution rate is generally comprised between 0,005 and 0,1 h⁻¹, preferably between 0,005 and 0,02 h⁻¹. The dilution rate can be changed during the adaptation method, eventually with a first step comprised between 0,005 and 0,02 h⁻¹ and a second step where the dilution rate is increased up to 0,1 h⁻¹, more preferably 0,06 h⁻¹

The microorganism adapted with the method of the present invention is preferably a producing microorganism being further adapted to grow in the presence of a high concentration of industrial glycerine.

The microorganism can also be a microorganism first adapted to grow in the presence of a high concentration of industrial glycerine further modified to have its glycerol metabolism is directed to 13,-propanediol production.

The "adapted microorganism" according to the invention is a producing microorganism adapted to grow in the presence of a high concentration of industrial glycerine and has two essential characteristics:
- its glycerol metabolism is directed to 13,-propanediol production, and
- it is allowed to grow in the presence of a high concentration of industrial glycerine

In one embodiment, the *C. acetobutylicum* DG1 pSPD5 strain is cultivated in continuous culture using a fed medium containing 105g.l. of raw glycerol from the biodiesel production, at a low dilution rate comprised between 0.005 and 0.02h-1, preferably 0.02h-1. Over a period of maximum 10 days, preferably between 5 and 8 days, the strain is adapted to the high glycerine concentration present in the fed medium, and the dilution rate can be increased up to 0.1 h⁻¹, preferably up to 0.06h-1 (see example 2).

The gradual increase of the dilution rate can be done between the end of the batch phase and 10 days, preferentially after 5 days, between 5 and 8 days, about 7 days, resulting in an improved productivity of the continuous culture (see example 3).

Advantageously, after the adaptation phase, the glycerol concentration in the fed medium can be increased up to 120g.l. However, direct attempt to adapt the strain to 120g.l. of industrial glycerol is not possible, even at a dilution rate of 0.02h-1 as for a glycerol concentration of 105g.l., again demonstrating that the strain is not able to grow in the presence of high glycerine concentration in the fed medium without prior adaptation.

### Examples

### Example 1

The synthetic media used for *Clostridium* batch cultivations contained per litre of de-ionized water: glycerol, 30g; KH2PO4, 0.5; K2HPO4, 0.5g; MgSO4, 7H2O, 0.2g; CoCl2 6H2O, 0.01g, H2SO4, 0.1ml; NH4CI, 1.5g, biotin, 0.16mg; p-amino benzoic acid, 32mg and FeSO4, 7H2O, 0.028g. The pH of the medium was adjusted to 6.3 with NH4OH 3N. For batch cultivation commercial glycerol purchased from Sigma (purity 99.5%) was used. The feed medium for continuous cultures contained par litre of tap water: raw glycerol, 105g; KH2PO4, 0.5; K2HPO4, 0.5g; MgSO4, 7H2O, 0.2g; CoCl2 6H2O, 0.026g; NH4CI, 1.5g, biotin, 0.16mg; p-amino benzoic acid, 32mg; FeSO4, 7H2O, 0.04g, antifoam, 0,05ml; ZnSO4, 7H2O, 8mg; CuCl2, 2H2O, 4mg; MnSO4, H2O, 40mg, H3BO3, 2mg; Na2MoO4 2H2O, 0.8mg. Medium pH was not adjusted in this case. Raw glycerol resulting from the trans-esterification process of biodiesel production was supplied by SAIPOL (Le Meriot, France) and contained 83%glycerol (w/w).

Continuous cultures were performed in a 2L bioreactor Tryton (Pierre Guerin, France) with a working volume of 1000ml. The culture volume was kept constant at 1000ml by automatic regulation of the culture level. Cultures were stirred at 200 RPM, at a temperature of 35°C and pH was maintained at 6.5 by automatic addition of NH4OH 3N. The Oxido-Reductive Power of the culture medium expressed in mV, was controlled and recorded during the experiment. To create anaerobic conditions, the sterilized medium in the vessel was flushed with sterile O2-free nitrogen for one hour at 60°C and flushed again until 35°C was reached. The bioreactor gas outlet was protected form the oxygen by a pyrogallol arrangement (Vasconcelos *et al.,* 1994). After sterilisation the feed medium was also flushed with sterile O2-free nitrogen until room temperature was attained and maintained under nitrogen pressure at 200mbar to avoid O2 entry.

Cell concentration was measured turbidimetrically at 620nm and correlated with cell dry weight evaluated directly. Glycerol, 1,3-propanediol, ethanol, butanol, acetic and butyric acids and others trace levels acids concentrations were measured by HPLC analysis. Separation was performed on a Biorad Aminex HPX-87H column and detection was achieved by refractive index. Operating conditions were as follows: mobile phase sulphuric acid 0.5mM; flow rate 0.5ml/min, temperature, 25°C.

A growing culture in 100ml flasks on synthetic medium (the same as the batch culture medium described above but with the addition of acetic acid, 2.2g.1-1 and MOPS, 23.03g.1-1) taken at the end of exponential growth phase was used as inoculums (5% v/v). The cultures were first grown batch-wise. At the early exponential growth phase a pulse of commercial glycerol was added: the pulse is defined by an addition of synthetic medium (the same as described for batch culture) with commercial glycerol 120g.l-1 at a flow rate of 50ml.h-1 during 3 hours (*i.e.* an addition of 18g of glycerol). Then the growth continued batch-wise and before the end of the exponential growth phase the continuous feeding started with a dilution rate of 0.06h-1 and a feed medium containing 105g.1-1 of raw glycerol.

**Table 1: Continuous culture of C. acetobutylicum DG1 (pSPD5) on raw glycerol at 105 g.l-1 (D = 0.06h-1, pH 6.5 ant T°C = 35°C). The given values represent an average of 7 points in steady state condition obtained after 3 volume changes.**

| | Average and |
|---|---|
| | standard deviation |
| Feed glycerol (g.l⁻¹) | 106.1 +/- 0.00 |
| 1,3-propanediol (g.l⁻¹) | 32.68 +/- 1.16 |
| Y₁,_{3-PDO} (9.9⁻¹) | 0.50 +/- 0.02 |
| Q_{1,3PDO} (g.l⁻¹.h⁻¹) | 2.00 +/- 0.09 |
| | |
| Dilution rate (h⁻¹) | 0.061 +/- 0.003 |
| Residual glycerol (g.l⁻¹) | 36.38 +/- 1.88 |
| Biomass (g.l⁻¹) | 1.83 +/- 0.09 |
| Acetic acid (g.l⁻¹) | 0.99 +/- 0.07 |
| Butyric acid (g.1⁻¹) | 7.65 +/- 0.44 |
| Carbon recovery (%) | 96.9 +/- 2.5 |

| | |
|---|---|
| Y1,3-PDO : PDO yield (g/g of glycerol consumed) Q1,3PDO : PDO volumetric productivity For the carbon recovery calculation, carbon dioxide concentration was estimated from end-products concentrations. | |

### Example 2

The synthetic media used were the same as described in example 1. Pre-culture, Inoculation and batch-wise growth were performed in the same conditions as described above.

The continuous feeding started with a dilution rate of 0.02h-1 and a feed medium containing 105g.l-1 of raw glycerol. After few days in these conditions (*i.e*. 8-10 days after inoculation of the bioreactor corresponding to 3 volumes changes at least) the dilution rate was increased from 0.02h-1 to 0.05h-1 according to the following scheme: i) increase of 0.01h-1 unit in 48 hours and ii) resting step of 24-48 hours, repeated 3 times. Stability of the culture was followed by products analysis using the HPLC protocol previously described. Notably, we waited for residual glycerol to be as low as possible to make a final increased of the dilution rate to 0.06h-1 in 48 hours.

**Table 2: Continuous culture of C. acetobutylicum DG1 (pSPD5) on raw glycerol at 105 g.l-1 (D = 0.05h-1 and 0.06h-1, pH 6.5 ant T°C = 35°C). The given values represent an average of 3 or 4 points in steady state condition.**

| | Average and | Average and standard |
|---|---|---|
| | standard deviation | deviation for D = |
| | for D = 0,05h⁻¹ | 0,06h⁻¹ |
| Feed glycerol (g.l⁻¹) | 108.36 +/- 0.00 | 110.01 +/- 0.00 |
| 1,3-propanediol (g.l⁻¹) | 51.92 +/- 0.29 | 50.95 +/- 0.52 |
| Y₁,_{3-PDO} (9.9⁻¹) | 0.53 +/- 0.00 | 0.51 +/- 0.01 |
| Q_{1,3PDO} (g.l⁻¹.h⁻¹) | 2.68 +/- 0.04 | 2.87 +/- 0.02 |
| | | |
| Dilution rate (h⁻¹) | 0.052 +/- 0.001 | 0.056 +/- 0.000 |
| Residual glycerol (g.l⁻¹) | 3.66 +/- 0.40 | 4.51 +/- 0.33 |
| Biomass (g.l⁻¹) | 1.94 +/- 0.07 | DO |
| Acetic acid (g.l⁻¹) | 3.27 +/- 0.11 | 2.67 +/- 0.06 |
| Butyric acid (g.l⁻¹) | 10.75 +/- 0.44 | 10.97 +/- 0.21 |
| Carbon recovery (%) | 97.4 +/- 1.4 | 95.1 +/- 1.2 |

| | | |
|---|---|---|
| Y1,3-PDO : PDO yield (g/g of glycerol consumed) Q1,3PDO : PDO volumetric productivity For the carbon recovery calculation, carbon dioxide concentration was estimated from end-products concentrations. | | |

Another culture performed in the same conditions led to the same results.

### Example 3

The synthetic media used were the same as described in example 1, except that the feed medium contained 120g.l-1 of raw glycerol. Pre-culture, Inoculation and batch-wise growth were performed in the same conditions as described above.
**A)** The continuous feeding started with a dilution rate of 0.02h-1 and a feed medium containing directly 120g.l-1 of raw glycerol.

**Table 3: Continuous culture of C. acetobutylicum DG1 (pSPD5) on raw glycerol at 120 g.l⁻¹ (D = 0.02h⁻¹, pH 6.5 ant T°C = 35°C). The given values represent an average of 7 points in steady state condition obtained after 3 volume changes.**

| | Average and |
|---|---|
| | standard |
| | deviation |
| Feed glycerol (g.l⁻¹) | 123.67 +/- 0.00 |
| 1,3-propanediol (g.l⁻¹) | 43.13 +/- 1.47 |
| Y₁,_{3-PDO} (g.g⁻¹) | 0.50 +/- 0.01 |
| Q_{1,3PDO} (g.l⁻¹.h⁻¹) | 1.01 +/- 0.05 |
| | |
| Dilution rate (h⁻¹) | 0.023 +/- 0.001 |
| Residual glycerol (g.l⁻¹) | 30.87 +/- 2.62 |
| Biomass (g.l¹) | 1.19 +/- 0.09 |
| Acetic acid (g.l⁻¹) | 2.36 +/- 0.18 |
| Butyric acid (g.l⁻¹) | 9.32 +/- 0.45 |
| Carbon recovery (%) | 94.2 +/- 1.4 |

| | |
|---|---|
| Y₁,_{3-PDO} : PDO yield (g/g of glycerol consumed) Q₁,_{3PDO} : PDO volumetric productivity For the carbon recovery calculation, carbon dioxide concentration was estimated from end-products concentrations. | |

**B)** The continuous feeding started with a dilution rate of 0.02h-1 and a feed medium containing first 105g.l-1 of raw glycerol. After few days in these conditions (corresponding to 3 volumes changes at least), the dilution rate was increased from 0.02h-1 to 0.06h-1 according to the scheme described previously. Then the feeding was switched to a dilution rate of 0.05h-1 and a feed medium containing 120g.l-1 of raw glycerol.

**Table 4: Continuous culture of C. acetobutylicum DG1 (pSPD5) on raw glycerol at 120 g.l⁻¹ (D = 0.05h⁻¹, pH 6.5 ant T°C = 35°C) after feeding at 105g.l⁻¹ raw glycerol at D = 0.06h⁻¹. The given values represent an average of 3 points obtained after at least 20 volume changes.**

| | Average and standard deviation |
|---|---|
| Feed glycerol (g.l⁻¹) | 123.68 +/- 0.00 |
| 1,3-propanediol (g.l⁻¹) | 53.53 +/- 0.59 |
| Y₁,_{3-PDO} (g.g⁻¹) | 0.53 +/- 0.02 |
| Q_{1,3PDO} (g.l⁻¹.h⁻¹) | 2.86 +/- 0.02 |
| | |
| Dilution rate (h⁻¹) | 0.053 +/- 0.000 |
| Residual glycerol (g.l⁻¹) | 15.58 +/- 3.56 |
| Biomass (g.l⁻¹) | 1.14 +/- 0.18 |
| Acetic acid (g.l⁻¹) | 4.30 +/- 0.10 |
| Butyric acid (g.l⁻¹) | 9.29 +/- 0.17 |
| Carbon recovery (%) | 94.8 +/- 2.3 |

| | |
|---|---|
| Y_{1,3-PDO} : PDO yield (g/g of glycerol consumed) Q_{1,3PDO} : PDO volumetric productivity For the carbon recovery calculation, carbon dioxide concentration was estimated from end-products concentrations. | |

### Cited documents

**1.** González-Pajuelo M, Meynial-Salles I, Mendes F, Andrade JC, Vasconcelos I, and Soucaille P. 2005. Metabolic engineering of Clostridium acetobutylicum for the industrial production of 1,3-propanediol from glycerol. Metabolic Engineering 7: 329-336.
**2.** González-Pajuelo M, Meynial-Salles I, Mendes F, Soucaille P. and Vasconcelos I. 2006. Microbial conversion of a natural producer, Clostridium butyricum VPI 3266, and an engineered strain, Clostridium acetobutylicum DG (pSPD5). Applied and Environmental Microbiology, 72: 96-101.
**3.** Nakas JP, Schaedle M, Parkinson CM, Coonley CE, and Tanenbaum SW. 1983. System development for linked-fermentation products of solvents from algal biomass. Applied and Environmental Microbiology 46: 1017-1023
**4.** Papanikolaou S, Ruiz-Sanchez P, Pariset B, Blanchard F and Fick M. 2000. High production of 1,3-propanediol from industrial glycerol by a newly isolated Clostridium butyricum strain. Journal of Biotechnology. 77: 191-2008.
**5.** WO2006/128381. Method for preparing 1,3-propanediol by using glycerine as the by-product of the biological diesel oil.

## Claims

1. A method for the production of 1,3-propanediol in a continuous fermentation process of glycerine, comprising culturing a producing microorganism on a culture medium, said producing microorganism allowing the conversion of glycerol into 1,3-propanediol, and recovering the 1,3-propanediol, **characterized in that** the culture medium comprises a high concentration of industrial glycerine and wherein the producing microorganism is a microorganism previously adapted to grow in the presence of a high concentration of industrial glycerine.

2. The method of claim 1 **characterized in that** the glycerol concentration in the culture medium is comprised between 90 and 120 g/L glycerol, preferably about 105g/L of glycerol.

3. The method according to claim 2, **characterized in that** the industrial glycerine is a by-product from biodiesel production.

4. The method according to any one of claims 1 to 3, **characterized in that** the culture medium is a synthetic medium, without addition of organic nitrogen.

5. The method according to any one of claims 1 to 4, **characterized in that** the producing microorganism is selected from members of the genera *Clostridium.*

6. The method according to claim 5 **characterized in that** the producing microorganism is *Clostridium acetobutylicum.*

7. The method according to any one of claims 1 to 6, **characterized in that** the producing microorganism is a genetically modified bacteria

8. The method according to any one of claims 1 to 7, **characterized in that** the glycerol dehydratase activity in the producing microorganism is independent of the presence of coenzyme B12 or one of its precursors and is derived from *Clostridium butyricum.*

9. The method according to any one of claims 1 to 8, **characterized in that** the microorganism is adapted into a producing microorganism by culturing the microorganism on a culture medium comprising high concentration of industrial glycerine at a low dilution rate and selecting the adapted microorganism able to grow on the culture medium having high concentration of industrial glycerine.

10. The method according to any one of claims 1 to 9, **characterized in that** the 1,3-propanediol is further purified.

11. A method for the modification of a microorganism into a microorganism adapted to grow in the presence of a high concentration of industrial glycerine, comprising culturing the microorganism on a culture medium comprising high concentration of industrial glycerine at a low dilution rate and selecting the adapted microorganism able to grow on the culture medium having high concentration of industrial glycerine.

12. The method of claim 11, **characterized in that** the microorganism is cultured at low dilution rate over a period ranging from 24 hours to 10 days.

13. The method of one of claims 11 or 12, **characterized in that** the dilution rate is comprised between 0,005 and 0,1 h⁻¹.

14. A microorganism adapted to grow in the presence of a high concentration of industrial glycerine obtainable by the method as claimed in one of claims 11 to 13.
